# EUROPEAN PATENT APPLICATION

(11) **EP 4 488 691 A1**
(43) Date of publication of application: **08.01.2025**
(21) Application number: 23760223.0
(22) Date of filing: 28.02.2023
(51) Int. Cl.: G01N 35/08, B01J 19/00, B81B 1/00, B81C 1/00, G01N 37/00

(54) **LIQUID SAMPLE ANALYSIS MICROCHIP AND METHOD FOR MANUFACTURING SAME**

(30) Priority: 28.02.2022 JP 2022029055
(71) Applicant: Fujimori Kogyo Co., Ltd., Tokyo 112-0002 (JP)
(72) Inventor: ABE, Masato, Tokyo 112-0002 (JP); HOSOKAWA, Kazuya, Tokyo 112-0002 (JP)
(74) Representative: Jones, Nicholas Andrew
(86) International application number: PCT/JP2023/007385
(87) International publication number: WO 2023/163225

(57) **Abstract**

A microchip for analyzing a liquid sample by allowing the liquid sample to pass from a liquid introduction portion to a channel is formed by attaching a film and a substrate of which a surface is provided with a penetrating hole serving as the liquid introduction portion and a groove serving as the channel, to each other. In the microchip, at least a part of a portion corresponding to the channel is subjected to hydrophilization treatment on a substrate-bonding surface of the film.

## Description

### Technical Field

The present invention relates to a microchip for analyzing a liquid sample and a method of producing the microchip.

### Background Art

It is known that a liquid sample such as blood is introduced into a channel in a microchip and reacted with a reagent in the channel to analyze a component in the liquid sample. In such an analysis method, a microchip produced by a method in which a film and a substrate on whose surface a groove serving as a channel is formed are attached to each other with an adhesive agent (for example, Patent Literature 1) can be used. However, use of a pump or the like is required for introducing a liquid sample into the channel in the microchip. A microchip in which passage of a liquid can be easily controlled without using a pump or the like has been demanded for enabling simpler measurement.

### Citation List

### Patent Literature

| | |
|---|---|
| Patent Literature 1 | JP2007-240461 A |

### Summary of Invention

### Technical Problem

An object of the present invention is to provide a microchip for analyzing a liquid sample by allowing the liquid sample to pass through an internal channel, and passage of a liquid can be easily controlled without using power such as a pump.

### Solution to Problem

In order to solve the above-described problem, the present inventors carried out an intensive study. As a result, it was found that a microchip can be easily produced by preparing a substrate including a bonding surface provided with a penetrating hole serving as a liquid introduction portion and a groove that forms a channel, applying an adhesive agent or a gluing agent to a region, excluding the penetrating hole and the groove, of the bonding surface, preparing a film that is to be attached to the bonding surface of the substrate, and in which at least a part of a portion corresponding to the channel is subjected to hydrophilization treatment, and attaching the film to the bonding surface of the substrate so that the groove of the substrate is covered with the film to form the channel, and a liquid can be passed into the channel only by adding a liquid sample to the liquid introduction portion in the obtained microchip. Further, it was found that passage of the liquid sample can be stopped by hydrophobization treatment of a portion of the channel of the bonding surface of the film, and passage of the liquid sample can be restarted by finger pressure or the like.

In other words, a first aspect of the present invention is a microchip for analyzing a liquid sample by allowing the liquid sample to pass from a liquid introduction portion to a channel, the microchip being formed by attaching a film and a substrate of which a surface is provided with a penetrating hole serving as the liquid introduction portion and a groove serving as the channel, to each other, wherein at least a part of a portion corresponding to the channel is subjected to hydrophilization treatment on a substrate-bonding surface of the film.

The microchip of this aspect can be produced by, for example, a method including:
a step of preparing a substrate including a bonding surface provided with a groove that forms a channel and a penetrating hole serving as a liquid introduction portion;
a step of applying an adhesive agent or a gluing agent to a region, excluding the groove and the penetrating hole, of the bonding surface;
a step of preparing a film that is to be attached to the bonding surface of the substrate, and in which at least a part of a portion corresponding to the channel has been subjected to hydrophilization treatment; and
a step of attaching the film to the bonding surface of the substrate so that the groove of the substrate is covered with the film to form the channel.

A second aspect of the present invention is a microchip for analyzing a liquid sample by allowing the liquid sample to pass from a liquid introduction portion to a channel, the microchip being formed by attaching a film and a substrate of which a surface is provided with a penetrating hole serving as the liquid introduction portion and a groove serving as the channel, to each other, wherein, on a substrate-bonding surface of the film, a part of a portion corresponding to the channel is subjected to hydrophilization treatment and another part of the portion corresponding to the channel is subjected to hydrophobization treatment.

The microchip of this aspect can be produced by, for example, a method including:
a step of preparing a substrate including a bonding surface provided with a groove that forms a channel and a penetrating hole serving as a liquid introduction portion;
a step of applying an adhesive agent or a gluing agent to a region, excluding the groove and the penetrating hole, of the bonding surface;
a step of preparing a film that is to be attached to the bonding surface of the substrate, and in which a part of a portion corresponding to the channel has been subjected to hydrophilization treatment and another part of the portion corresponding to the channel has been subjected to hydrophobization treatment; and
a step of attaching the film to the bonding surface of the substrate so that the groove of the substrate is covered with the film to form the channel.

Moreover, in this case, the slight presence of an untreated portion subjected to neither the hydrophilization treatment nor the hydrophobization treatment between the portion subjected to the hydrophilization treatment and the portion subjected to the hydrophobization treatment causes no problem.

A third aspect of the present invention is a microchip for analyzing a liquid sample by allowing the liquid sample to pass from a liquid introduction portion to a channel, the microchip being formed by attaching a film and a substrate of which a surface is provided with a penetrating hole serving as the liquid introduction portion, a groove serving as the channel, and a recess serving as a reaction portion disposed in a portion of the groove, to each other, wherein, on a substrate-bonding surface of the film, a reagent is coated on a portion corresponding to the reaction portion, a portion corresponding to a starting end of a downstream channel that comes into contact with the reagent-coated portion is subjected to hydrophobization treatment, and the remaining part of the portion corresponding to the channel is subjected to hydrophilization treatment.

The microchip of this aspect can be produced by, for example, a method including:
a step of preparing a substrate including a bonding surface provided with a groove that forms a channel, a recess serving as a reaction portion disposed in a portion of the groove, and a penetrating hole serving as a liquid introduction portion;
a step of applying an adhesive agent or a gluing agent to a region, excluding the groove, the recess, and the penetrating hole, of the bonding surface;
a step of preparing a film that is to be attached to the bonding surface of the substrate, and in which a reagent has been coated on a portion corresponding to the reaction portion, a portion corresponding to a starting end of a downstream channel that comes into contact with the reagent-coated portion has been subjected to hydrophobization treatment, and the remaining part of the portion corresponding to the channel has been subjected to hydrophilization treatment; and
a step of attaching the film to the bonding surface of the substrate so that the groove of the substrate is covered with the film to form the channel.

Moreover, in this case, the slight presence of an untreated portion subjected to neither the hydrophilization treatment nor the hydrophobization treatment between the portion subjected to the hydrophilization treatment and the portion subjected to the hydrophobization treatment causes no problem.

### Advantageous Effects of Invention

According to the present invention, a microchip in which passage of a liquid can be easily controlled without using a power apparatus such as a pump.

### Brief Description of Drawings

Fig. 1 is a diagram illustrating the substrate of a microchip according to one aspect of the present invention.
Fig. 2 is a diagram illustrating the film of a microchip according to one aspect of the present invention.
Fig. 3 is a diagram illustrating a completed microchip according to one aspect of the present invention.
Fig. 4 is a diagram illustrating a use aspect (state of flow of liquid sample) of a microchip according to one aspect of the present invention.
Fig. 5 is a diagram illustrating the film of a microchip according to another aspect of the present invention.
Fig. 6 is a diagram illustrating a completed microchip according to another aspect of the present invention.
Fig. 7 is a diagram illustrating a use aspect (state of introduction of liquid sample) of a microchip according to another aspect of the present invention.
Fig. 8 is a diagram illustrating a use aspect (state of flow of liquid sample) of a microchip according to another aspect of the present invention.
Fig. 9 is a diagram illustrating the substrate of a microchip including a reaction portion according to another aspect of the present invention.
Fig. 10 is a diagram illustrating the film of a microchip including a reaction portion according to another aspect of the present invention.
Fig. 11 is a diagram illustrating a completed microchip including a reaction portion according to another aspect of the present invention.

### Description of Embodiments

A microchip of the present invention is a microchip for analyzing a liquid sample by allowing the liquid sample to pass from a liquid introduction portion to a channel, the microchip being formed by attaching a film and a substrate of which a surface is provided with a penetrating hole serving as the liquid introduction portion and a groove serving as the channel, to each other, wherein at least a part of a portion corresponding to the channel is subjected to hydrophilization treatment on a substrate-attaching surface of the film. The substrate preferably further includes a penetrating hole serving as an outlet in the end of the side, different from the side of the penetrating hole serving as the liquid introduction portion, of the groove serving as the channel.

Such a liquid sample is not particularly restricted as long as the sample can be passed into the microchip, and examples thereof include a liquid sample obtained from a living body, such as blood or urine, or a diluted liquid thereof, an extract from a living body, such as a plant or animal, naturally occurring water, such as river, ocean, or rainfall, washing liquid, and waste liquid. A component in the sample is not particularly restricted, and examples thereof include a protein, a nucleic acid, a low molecular weight compound, and a sugar.

The liquid sample is preferably an aqueous solution or an aqueous dispersion. The aqueous dispersion is in a state in which a substance, a cell, a blood cell, or the like is dispersed in a liquid mainly containing water.

Analysis of a liquid sample using the microchip of the present invention may be analysis of a liquid property such as a passage rate in a channel of a liquid sample, and is preferably analysis of a component in the sample by allowing the liquid sample to react with a reactant mounted in a portion of the channel.

Hereinafter, the microchip for analyzing a liquid sample of the present invention and the method of producing the microchip will be described with reference to the drawings. However, the following is only an example, and the manufacturing method of the present invention and a microchip obtained by the method are not limited to the following aspects.

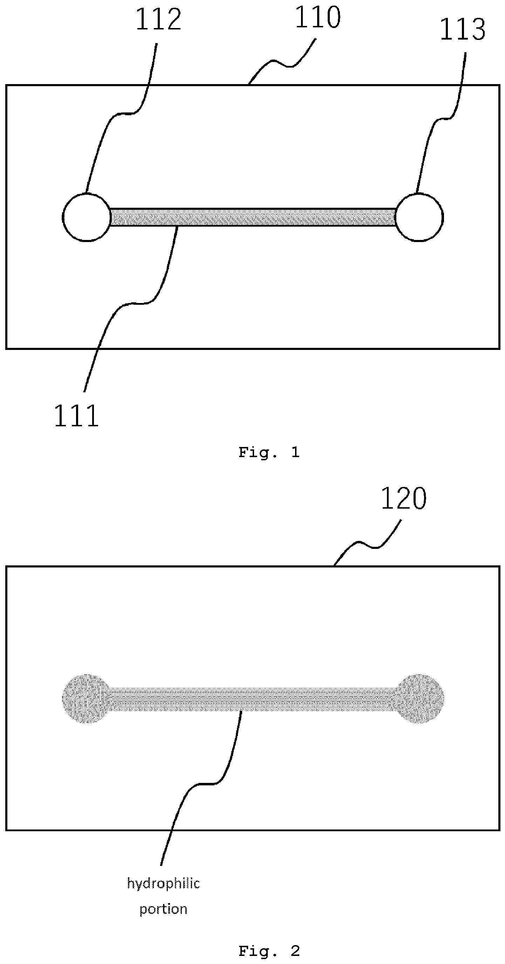

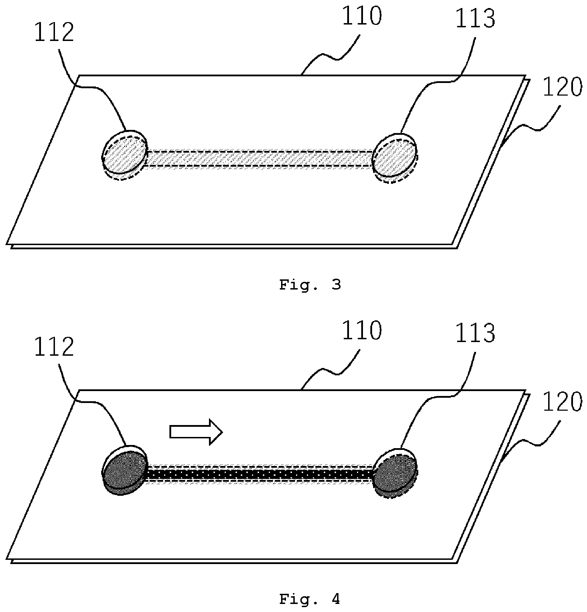

Fig. 1 is a diagram illustrating the substrate 110 of a microchip 100. Fig. 2 is a diagram illustrating the film 120 of the microchip 100. Fig. 3 is a diagram illustrating the completed microchip 100 in which the film 120 is attached to the substrate 110.

### <Substrate>

Fig. 1 is a plan view of the substrate 110 on the surface of which a groove 111 that forms the channel of the microchip 100 is formed. The surface on which the groove 111 is formed is a film 120-attaching surface, and is also referred to as a bonding surface.

One end of the groove 111 is provided with a penetrating hole serving as a liquid introduction portion (inlet) 112 for a liquid sample, and the other end side is provided with a penetrating hole serving as an outlet 113 (exhaust port). A recess serving as a waste liquid storage portion for internally storing a waste liquid may be disposed instead of the penetrating hole serving as the outlet.

Two or more channels 111 may be disposed. The shape of the channel 111 may be any shape, and may be straight or curved. The channel 111 may include a branch. In such a case, two or more liquid introduction portions 112 and/or outlets 113 may exist. For example, it is possible that two liquid introduction portions 112 are disposed, a liquid sample is allowed to flow from a first inlet into a first channel, and a reaction substrate liquid is allowed to flow from a second inlet into a second channel.

The cross-sectional shape of the groove 111 serving as the channel may be any shape, such as concave, U-shaped, or V-shaped. The depth of the groove 111 serving as the channel is preferably from 10 to 500 µm, and the width of the groove is preferably from 10 µm to 3 mm.

The size of a penetrating hole serving as the liquid introduction portion 112 may be any size that allows injection of a liquid sample such as blood using a micro syringe or the like. The size of the penetrating hole serving as the liquid introduction portion 112 is, for example, a diameter of 0.2 to 3 mm. The size of a penetrating hole serving as the outlet 113 is not particularly restricted, as long as the penetrating hole is large enough to function as an outlet for a liquid sample. The size of the penetrating hole serving as the outlet 113 is, for example, a diameter of 0.2 to 2 mm.

Metal, glass, plastic, silicone, or the like can be used as the material of the microchip 100, and from the viewpoint of detecting a reaction by luminescence, coloration, or visual inspection, a transparent material is preferable, and a transparent plastic is more preferable. Examples of the material of the microchip 100 include polyethylene, polypropylene, polystyrene, polymethyl methacrylate, cycloolefin polymer, cycloolefin copolymer, polyphenylene oxide, polyethylene terephthalate, polyethylene naphthalate, polycarbonate, polyamide, polyimide, a phenol resin, an epoxy resin, a polyvinylidene chloride, a polyvinyl chloride, an ABS resin, and a poly(2-methoxyethyl acrylate) (PMEA) resin.

The groove 111 or a penetrating hole disposed in the substrate 110 of the microchip 100 can be formed with a blade or a laser beam, and when the material of the microchip 100 is plastic, the groove or penetrating hole can also be formed by injection molding. Formation by injection molding is preferable since the microchip 100 of consistent quality can be produced efficiently.

Fig. 2 is a plan view of the film 120 of the microchip 100.

A portion corresponding to a channel in a substrate-attaching surface (bonding surface) of the film 120 is subjected to hydrophilization treatment.

A wide region in a range covering a channel when the film is bonded to the substrate may also be subjected to the hydrophilization treatment.

It is preferable to also perform hydrophilization treatment of a region corresponding to the liquid introduction portion and a region corresponding to the outlet in the bonding surface of the film.

### <Film>

The material of the film 120 is preferably a transparent plastic, and the materials described above are exemplified, and a polyethylene terephthalate (PET) resin, a cycloolefin polymer (COP) resin, a cycloolefin copolymer (COC) resin, a polystyrene (PS) resin, a polycarbonate (PC) resin, or a polymethyl methacrylate (PMMA) resin is more preferable. The thickness of the film 120 is, for example, preferably from 50 to 200 µm, and more preferably from 100 to 200 µm.

The hydrophilization treatment is preferably performed by applying a hydrophilization reagent, a plasma treatment, or an excimer treatment. Examples of the hydrophilization reagent include a nonionic surfactant such as S-1570 (sucrose fatty acid esters: MITSUBISHI-CHEMICAL FOODS CORPORATION), LWA-1570 (sucrose laurate: MITSUBISHI-CHEMICAL FOODS CORPORATION), POEM DL-100 (diglycerin monolaurate: RIKEN VITAMIN Co., Ltd.), or RIKEMAL A (sucrose fatty acid esters: RIKEN VITAMIN Co., Ltd.), CeraAqua NS235-N1 (SHIMA TRADING CO., LTD.), Aminoion (NIPPON NYUKAZAI CO., LTD.), LAMBIC-771W (OSAKA ORGANIC CHEMICAL INDUSTRY LTD.), LAMBIC-1000W (OSAKA ORGANIC CHEMICAL INDUSTRY LTD.), SPRA-101 (TOKYO OHKA KOGYO CO., LTD.), and SPRA-202 (TOKYO OHKA KOGYO CO., LTD.). Examples of specific conditions include a condition where the water contact angle of a substrate surface is, for example, 55° or less.

In order to attach the film 120 onto the substrate 110, an adhesive agent and/or a gluing agent are used. The adhesive agent is applied to a portion excluding the channel and penetrating hole of a substrate. When the substrate includes a recess serving as a reaction portion, the adhesive agent is applied to a portion excluding the channel, recess, and penetrating hole of the substrate.

Examples of the adhesive agent include a (meth)acrylic resin-based adhesive, a natural rubber adhesive, a urethane resin-based adhesive, an ethylene-vinyl acetate resin emulsion adhesive, an ethylene-vinyl acetate resin-based adhesive, an epoxy resin-based adhesive, a vinyl chloride resin solvent-based adhesive, a chloroprene rubber-based adhesive, a cyanoacrylate-based adhesive, a silicone-based adhesive, a styrene-butadiene rubber solvent-based adhesive, a nitrile rubber-based adhesive, a nitrocellulose-based adhesive, a phenolic resin-based adhesive, a modified silicone-based adhesive, a polyester-based adhesive, a polyamide-based adhesive, a polyimide-based adhesive, an olefin resin-based adhesive, a vinyl acetate resin emulsion-based adhesive, a polystyrene resin solvent-based adhesive, a polyvinyl alcohol-based adhesive, a polyvinyl pyrrolidone resin-based adhesive, a polyvinyl butyral-based adhesive, a polybenzimidazole adhesive, a polymethacrylate resin solvent-based adhesive, a melamine resin-based adhesive, a urea resin-based adhesive, and a resorcinol-based adhesive. One or more adhesive agents can be used singly, or two or more kinds thereof can be used in mixture.

In order to attach the film 120 onto the substrate 110, a gluing agent which is one kind of adhesive agents may be used. Examples of the gluing agent include a rubber-based gluing agent, a (meth)acrylic gluing agent, a silicone-based gluing agent, a urethane-based gluing agent, a vinyl alkyl ether-based gluing agent, a polyvinyl alcohol-based gluing agent, a polyvinyl pyrrolidone-based gluing agent, a polyacrylamide-based gluing agent, and a cellulose-based gluing agent. Such gluing agents may be used singly, or two or more kinds thereof may be used in mixture.

The adhesive agent or gluing agent is preferably light-curing (either radical reactive or cationic polymerization), and more preferably UV-curing. With a UV curing adhesive agent (including UV gluing agent), after an application step, irradiation with UV light quickly initiates a curing reaction, allowing bonding to take place. For the UV curing adhesive agent, for example, an acrylic UV curing adhesive agent such as UVX-8204 (manufactured by Denka Company Limited.), UVX-8400 (manufactured by Denka Company Limited.), SX-UV100A (manufactured by CEMEDINE CO., LTD.), SX-UV200 (manufactured by CEMEDINE CO., LTD.), BBX-UV300 (manufactured by CEMEDINE CO., LTD.), U-1340 (Chemitech Inc.), U-1455B (Chemitech Inc.), U-1558B (Chemitech Inc.), Aronix UV-3000 (TOAGOSEI CO., LTD.), TB3094 (ThreeBond Co., Ltd.), or Hitaroid 7975D (Hitachi Chemical Company, Ltd.) is more preferable. For the UV curing gluing agent, an acrylic UV curing gluing agent such as UV-3630ID80 (Mitsubishi Chemical Corporation), UX-3204 (Nippon Kayaku Co., Ltd.), or FINETAC RX-104 (DIC Corporation) is more preferable. An acrylic UV curing adhesive agent and gluing agent can exhibit favorable adhesion to a wide range of plastic materials and achieve rapid strength development after UV irradiation. The viscosity of an adhesive agent (including gluing agent) used for attaching the film 120 onto the substrate 110 is preferably, for example, from 2,000 to 31,000 mPa·s.

For more accurately applying an adhesive agent (hereinafter description is provided with an adhesive agent, but the same applies in the case of a gluing agent) to a predetermined application region, the adhesive agent is preferably applied by printing technique, and particularly preferably applied by screen printing. By using screen printing, even when a screen printing plate in a region corresponding to the entire surface of the substrate 110 is filled with the adhesive agent, the adhesive agent is transferred to a predetermined application region that comes into contact with the screen printing plate, but the adhesive agent is not transferred to the groove 111 that does not come into contact with the screen printing plate, and an inlet and an outlet. Thus, the adhesive agent can be favorably applied to a predetermined application region.

The film thickness of the adhesive agent that is applied to the substrate 110 is preferably from 5 to 15 µm. For controlling the film thickness of the adhesive agent, the mesh count per inch of screen is preferably, for example, from 500 to 730. The opening ratio of the mesh is preferably, for example, from 39 to 47%. The thickness of a mesh is preferably, for example, from 15 to 28 µm. With this, the film thickness of the applied adhesive agent is preferably from 5 to 15 µm.

As other methods of applying an adhesive agent to the substrate 110, inkjet printing, gravure printing, or a dispenser can be used to precisely apply the adhesive agent to a predetermined application region. In these application techniques, when an adhesive agent is discharged against the groove 111, the adhesive agent applied inside the groove 111 changes the shape of the channel 111. Therefore, printing treatment and the operation of the dispenser may be controlled by a program so that an adhesive agent is applied to a predetermined application region by capturing an image of the position of the groove 111 of the substrate 110 or fixing a printing stage and the position of the substrate 110.

An adhesive agent may be applied after hydrophilization treatment of the bonding surface of the substrate 110. The hydrophilization treatment is preferably a plasma treatment, a corona treatment, or an excimer treatment. The substrate 110 and the film 120 can be favorably attached to each other by performing the hydrophilization treatment so that the substrate 110 does not repel the adhesive agent, and the adhesive agent spreads on the substrate 110 and does not flow into the groove 111.

### <Attachment Step>

Fig. 3 is a diagram illustrating a completed microchip in which a film is attached to a substrate. The channel 111 formed in the interior of the microchip 100 is indicated by the dashed lines.

By layering the film 120 on the substrate 110 and attaching the film 120 and the substrate 110 to each other to direct a film surface downward, a lower portion of the groove 111 is covered with the film 120, a gap between the film 120 and the groove 111 of the substrate is formed, and a channel including a hydrophilization-treated bottom surface through which a liquid passes is formed.

By layering the film 120, a side, closer to the bonding surface, of a penetrating hole of the substrate 110 is sealed, and only a surface reverse to the bonding surface serves as an opening. This allows the penetrating hole of the substrate 110 to function as the liquid introduction portion (inlet) 112 and the outlet 113.

Fig. 4 illustrates a use aspect of a microchip. A liquid sample introduced from a liquid introduction portion 112 flows into a channel, and immediately passes over a hydrophilization-treated surface of a film. The liquid sample can be analyzed by measuring the passage rate or the like thereof. Alternatively, a reagent that reacts with a component in the sample may be placed in the region of a portion of the channel closer to the film, and the component in the liquid sample may be analyzed by the reaction with the reagent.

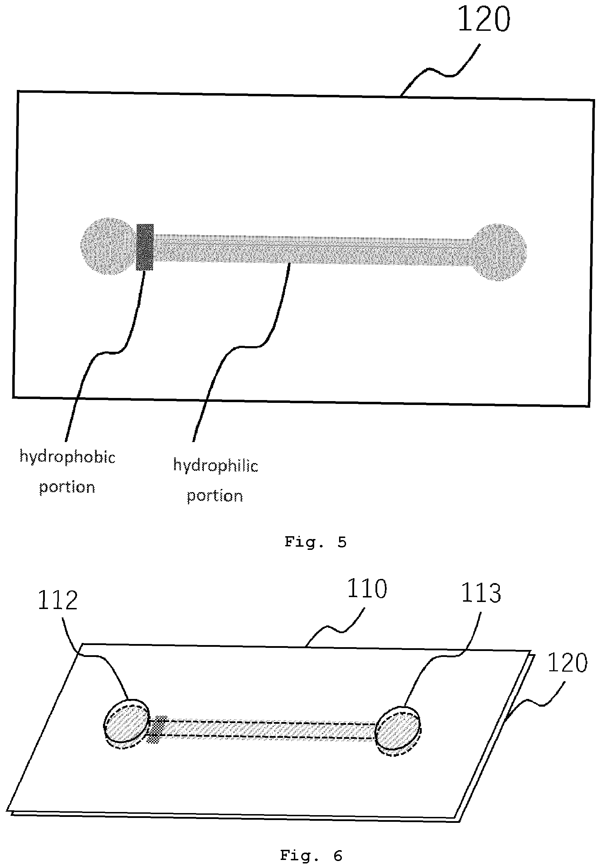

An aspect of a microchip in which a portion of a region corresponding to a channel on a substrate-bonding surface of a film is subjected to hydrophobization treatment, and the other portion is subjected to hydrophilization treatment is described with reference to Figs. 5 to 8. In this aspect, a portion corresponding to the starting end of the channel (portion that comes into contact with liquid introduction portion of channel) is subjected to hydrophobization treatment, and a portion corresponding to the terminal end of the channel (portion that comes into contact with outlet) or a portion corresponding to the middle of the channel may be subjected to hydrophobization treatment.

In this aspect, the substrate similar to the substrate in Fig. 1 can also be used.

In this aspect, a portion corresponding to the starting end of the channel of the film 120 is subjected to hydrophobization treatment, and the following portion is subjected to hydrophilization treatment, as illustrated in Fig. 4.

The hydrophilization treatment can be performed in a manner similar to the manner described above.

The hydrophobization treatment is preferably fluorine membrane coating by application or plasma treatment of a hydrophobization reagent. Examples of the hydrophobization reagent include FLUOROSURF FS-1610C-4.0 (FluoroTechnology Co., LTD.), water-repellent oil-repellent fluorine coating agent GF03 (GAST JAPAN Co., Ltd.), Guard Surf AZ-6000 (Harves Co., Ltd.), and fluorine coating agent INT series (Noda Screen Co., Ltd.). Examples of specific conditions include a condition where the water contact angle of the end surface of the wall is, for example, 110° or more.

A method of coating with a hydrophobization reagent is not particularly restricted, and a casting method enables precise application to a predetermined region by using an apparatus such as an inkjet or a dispenser, and can be preferably used for hydrophobization treatment of a micro region such as a portion corresponding to the middle of a channel.

For example, natural drying, vacuum drying, or warm air drying can be utilized as a method of drying a hydrophobization reagent after coating, but the method is not particularly limited.

By layering the film 120 on the substrate 110 to which an adhesive agent is applied, the microchip in which a boundary portion between the liquid introduction portion and the channel is subjected to hydrophobization treatment can be obtained (Fig. 6).

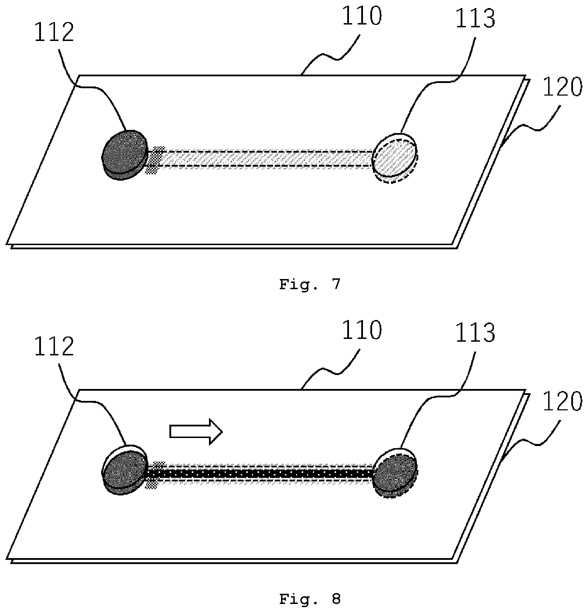

As illustrated in Fig. 7, a liquid sample is stored in the liquid introduction portion due to the presence of the hydrophobization-treated portion by adding the liquid sample to the liquid introduction portion of the microchip. By pressing the upper portion of the liquid introduction portion with a finger or the like, or pressurizing the upper portion using a pump or the like, the liquid sample in the liquid introduction portion is allowed to pass through a hydrophobic portion and pass through the hydrophilization-treated channel, as illustrated in Fig. 8. The liquid sample can be analyzed by measuring a passage rate or the like in such a case. Alternatively, a reagent that reacts with a component in the sample may be placed in the region of a portion of the channel of the film, and the component in the liquid sample may be analyzed by the reaction with the reagent. A method of passing a hydrophobic portion through a hydrophobic portion is not limited to pressurization, and, for example, centrifugal force can also be used for the method.

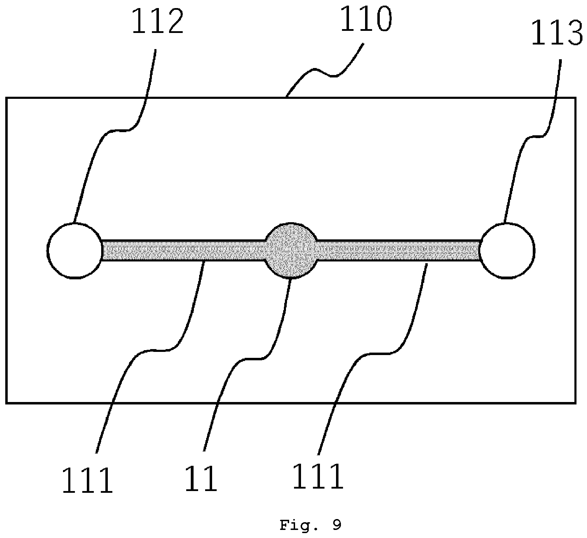

An aspect of a microchip in which a substrate includes a recess serving as a reaction portion in a portion of a channel, a film includes a reagent coated portion in a region corresponding to the reaction portion in a substrate-bonding surface of the film, a portion corresponding to the starting end of a downstream channel that comes into contact with the reagent coated portion is subjected to hydrophobization treatment, and a portion corresponding to the remaining part of the region of the channel is subjected to hydrophilization treatment is described with reference to Figs. 9 to 11.

As illustrated in Fig. 9, in this aspect, a substrate 110 includes a penetrating hole 112 serving as a liquid introduction portion, a groove 111 serving as a channel, and a penetrating hole 113 serving as an outlet, and in addition, a portion of the groove serving as the channel is provided with a recess 11 serving as a reaction portion. The depth of the recess may be equivalent to or more than the depth of the channel. The area thereof is not particularly restricted as long as the area enables a liquid sample to be stored for a reaction portion.

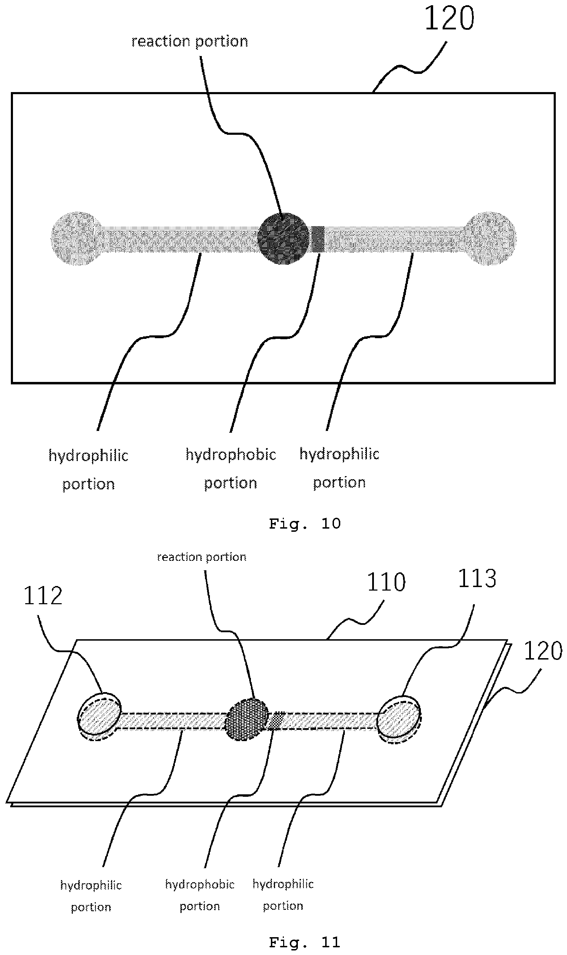

As illustrated in Fig. 10, in this aspect, in a film 120, portions corresponding to the channel (portions corresponding to an upstream channel portion and a downstream channel portion) are subjected to hydrophilization treatment, and a reagent mounting portion on which a reaction reagent is mounted is included in the middle of the channel. A portion that comes into contact with the reagent coated portion of the downstream channel portion is subjected to hydrophobization treatment. In such a case, the upstream channel portion means a channel from the liquid introduction portion to the reaction portion, and the downstream channel portion means a channel from the reaction portion to the outlet.

The reaction reagent can be any substance that reacts with a target (detection target) component in a liquid sample, and can be appropriately selected according to the type of a target substance. Examples of the reactivity of a reactive substance include a biological reaction and a chemical reaction, and examples of the biological reaction include a binding reaction. Examples of the reaction reagent include a protein (including a peptide), a sugar, a nucleic acid, and a low molecular weight compound. Examples thereof include a substance such as an antibody that binds specifically to a target substance, an enzyme protein that uses a target substance as a substrate, and a blood coagulation factor such as a PT reagent. When the target substance is a nucleic acid, a nucleic acid probe or a polymerase (nucleic acid amplifying enzyme) that amplifies a nucleic acid may be used.

The reaction reagent may be coated on or immobilized to the film. It is preferably that the reagent coated portion is also subjected to hydrophilization treatment.

By layering the film 120 on the substrate 110, the microchip in which the reaction reagent is mounted on the reaction portion, the upstream channel and the downstream channel are subjected to hydrophilization treatment, and a boundary portion between the reaction portion and the downstream channel is subjected to hydrophobization treatment can be obtained (Fig. 11).

By adding a liquid sample to the liquid introduction portion of the microchip, the liquid sample passes from the liquid introduction portion through the upstream channel portion and arrives at the reaction portion. In such a case, the presence of a hydrophobic treatment portion present in the boundary between the reaction portion and the downstream channel portion allows the liquid sample to be stored in the reaction portion and to react with the reaction reagent.

After the reaction, the upper portion of the liquid introduction portion is pressed with a finger or the like, and pressurized, to allow the liquid sample in the reaction portion to pass through the hydrophobic portion and to pass through the downstream channel subjected to hydrophilization treatment.

A target substance in a sample can be measured by observing or detecting a reaction in a reaction field, or a target substance in a sample discharged from the outlet 113 can also be measured. Examples of the reaction include, but are not limited to, a chromogenic reaction, a luminescence reaction, an amplification reaction, and an aggregation reaction.

Hereinafter, the present invention will be specifically described with reference to Examples, but the present invention is not limited to the following aspects.

### [Example 1]

### <Microchip Preparation 1>

A substrate 110 (injection molded product made of COP resin) (size of 59.4 × 26.2 mm, thickness of 3.0 mm) illustrated in Fig. 1 was prepared. In the substrate 110, a channel 111 had a length of 47.4 mm, a depth of 200 µm, and a width of 200 µm at an inlet. In the substrate 201, holes serving as an inlet 112 and an outlet 113 were a penetrating hole having an inner diameter of 2 mm and a circular cross sectional shape.

As a film 120, ZF14-100 (made of COP resin) (size of 59.4 × 26.2 mm, thickness of 100 µm) was prepared. The film 120 was surface-modified by corona treatment before application of a hydrophilization reagent so that a water contact angle was 40 to 50°, and the application property of the hydrophilization reagent was improved.

For hydrophilization treatment, 0.1% LWA-1570 aqueous solution was prepared as the hydrophilization reagent. The 0.1% LWA-1570 aqueous solution in an amount of 50 µL was applied to a substrate 110-bonding surface of the film 120. For an application region, it was applied to a region of 53 mm × 8 mm corresponding to the inlet 112, channel 111, and outlet 113 of the substrate 110 when the bonding was performed. Then, it was dried overnight at ordinary temperature and normal pressure to produce a hydrophilization region on the film 120.

An adhesive agent was used to attach the substrate 110 and the film 120 to each other. The adhesive agent was UVX-8204, a solvent-free, radical reactive acrylic UV curable adhesive agent. The adhesive agent was applied to a surface provided with the channel 111 of the substrate 110 by screen printing. In a screen plate used, the mesh count was 640, the opening ratio was 39%, and the application thickness of the adhesive agent was about 7 µm.

The adhesive agent applied to the surface of the substrate 110 was layered with the film 120 and irradiated with ultraviolet light having a wavelength of 365 nm for 10 to 20 seconds using a UV-LED light source to initiate a curing reaction of the adhesive agent and bond the film 120 onto the substrate 110.

### <Microchip Evaluation 1>

Into the inlet 112 of the produced microchip, 5 µL of distilled water was dripped to observe a liquid passage property. As a result of the experiment, distilled water was stored in the inlet 112 and liquid passage was not observed in the microchip produced without applying the hydrophilization reagent to the film 120, while passage of a liquid into the channel 111 due to a capillary phenomenon only by dripping distilled water was observed in the microchip produced by applying the hydrophilization reagent. The above results revealed that liquid passage occurred only due to dripping of the liquid sample into the inlet in the microchip produced by hydrophilization treatment of a region on the film corresponding to the channel.

### [Example 2]

### <Microchip Preparation 2>

A substrate and a film similar to those in Example 1 were used. Surface modification treatment of the film was performed by a method similar to the method in Example 1 before application of a hydrophilization reagent and a hydrophobization reagent.

For hydrophilization treatment of a film 120, 0.1% LWA-1570 aqueous solution was prepared as the hydrophilization reagent. The 0.1% LWA-1570 aqueous solution in an amount of 50 µL was applied to a substrate 110-bonding surface of the film 120. For an application region, it was applied to a region of 53 mm × 8 mm corresponding to the inlet 112, channel 111, and outlet 113 of the substrate 110 when the bonding was performed. In such a case, a hydrophilization reagent was not applied to a region having a width of 3 mm in a direction from the end of the inlet 112 to the outlet 113 on the substrate 110-bonding surface of the film 120.

For the hydrophobization treatment of the film 120, FLUOROSURF FS-1610C-4.0 was prepared as the hydrophobization reagent, and 2 µL of the hydrophobization reagent was dripped onto the film 120 so that the position thereof was adjusted with respect to a region having a width of 3 mm in the direction from the end of the inlet 112, to which the hydrophilization reagent had not been applied, to the outlet 113.

The film 120 to which the hydrophilization reagent and the hydrophobization reagent had been applied was dried overnight at ordinary temperature and normal pressure to obtain the film 120 as illustrated in Fig. 5.

The substrate 110 and the film 120 were bonded to each other by a method similar to the method in Example 1 to produce a microchip.

### <Microchip Evaluation 2>

Into the inlet 112 of the produced microchip, 5 µL of distilled water was dripped to observe a liquid passage property. As a result of the experiment, distilled water was uniformly filled in the inlet 112 in the microchip subjected to hydrophilization and hydrophobization treatment. However, since a portion just after the inlet 112 was subjected to hydrophobization treatment, liquid passage did not occur to the channel 111.

After filling the inlet 112 with distilled water, the inlet 112 was covered with a silicone sheet having a thickness of 3 mm, and a pressure was applied onto the silicone sheet with a finger. Thus, it was observed that distilled water passed through the hydrophobization region, and the distilled water that had jumped out to the channel 111 spread out to an outlet portion which was the other end of the hydrophilization region even after the end of the pressurization with the finger.

These results revealed that the starting and stopping of liquid passage in an optional region in a channel were able to be controlled by hydrophilization and hydrophobization treatment of a film.

A method of restarting the liquid passage of a liquid sample stopped in a hydrophobization region need not be a pressure, and, for example, a liquid sample can also be driven by centrifugal force by rotating a microchip using an apparatus.

### [Example 3]

### <Microchip Preparation 3>

A substrate 110 (injection molded product made of COP resin) (size of 59.4 × 26.2 mm, thickness of 3.0 mm) illustrated in Fig. 9 was prepared. In the substrate 110, a channel 111 had a length of 47.4 mm, a depth of 200 µm, and a width of 200 µm at an inlet. In the substrate 201, holes serving as an inlet 112 and an outlet 113 were a penetrating hole having an inner diameter of 2 mm and a circular cross sectional shape. The center of the channel 111 is provided with a reaction portion 11 having an inner diameter of 2 mm, a depth of 2.2 mm, and a circular cross section.

For hydrophilization treatment, 0.1% LWA-1570 aqueous solution was used as the hydrophilization reagent. The 0.1% LWA-1570 aqueous solution in an amount of 50 µL was applied to a substrate 110-bonding surface of a film 120. For an application region, it was applied to a region of 45.3 mm × 8 mm corresponding to the inlet 112, channel 111, reaction portion 11, and outlet 113 of the substrate 110 when the bonding was performed. In such a case, a hydrophilization reagent was not applied to a region having a width of 3 mm in a direction from the end of the reaction portion 11 to the outlet 113.

For hydrophobization treatment, FLUOROSURF FS-1610C-4.0 was prepared as a hydrophobization reagent, and 2 µL of the hydrophobization reagent was dripped onto the film 120 so that the position thereof was adjusted with respect to a region having a width of 3 mm in the direction from the end of the reaction portion 11, to which the hydrophilization reagent had not been applied, to the outlet 113.

The film 120 to which the hydrophilization reagent and the hydrophobization reagent had been applied was dried overnight at ordinary temperature and normal pressure.

An aqueous red dye was coated on a region corresponding to the reaction portion 11, and dried overnight at ordinary temperature and normal pressure to obtain the film 120 as in Fig. 10.

### <Microchip Evaluation 3>

Into the inlet 112 of the produced microchip, 5 µL of distilled water was dripped to observe a liquid passage property. As a result of the experiment, liquid passage into the channel 111 due to a capillary phenomenon only by dripping distilled water was observed in the produced microchip, and the liquid passage was stopped when the reaction portion 11 was filled. By filling the reaction portion 11 with the distilled water, the aqueous red dye coated on the film 120 was eluted, and the distilled water turned red. Then, the inlet 112 was covered with a silicone sheet having a thickness of 3 mm, and a pressure was applied onto the silicone sheet with a finger. Thus, distilled water passed through the hydrophobization region, and the distilled water that had passed through the hydrophobization region passed through the channel 111 and spread out to the outlet which was the other end of the hydrophilization region.

The above results revealed that the liquid sample was able to be stopped in the middle of the channel or the reaction portion, and liquid passage was able to be restarted at optional timing in the microchip produced by hydrophilization treatment of a region on the film corresponding to the channel.

### Reference Signs List

- 100: Microchip

- 110: Substrate
- 111: Channel (groove)
- 112: Liquid introduction portion (inlet)
- 113: Outlet
- 11: Reaction portion
- 120: Film

## Claims

1. A microchip for analyzing a liquid sample by allowing the liquid sample to pass from a liquid introduction portion to a channel, the microchip being formed by attaching a film and a substrate of which a surface is provided with a penetrating hole serving as the liquid introduction portion and a groove serving as the channel, to each other, wherein at least a part of a portion corresponding to the channel is subjected to hydrophilization treatment on a substrate-bonding surface of the film.

2. The microchip according to claim 1, wherein, on a substrate-bonding surface of the film, a part of the portion corresponding to the channel is subjected to hydrophilization treatment, and another part of the portion corresponding to the channel is subjected to hydrophobization treatment.

3. The microchip according to claim 1 or 2, wherein, on a substrate-bonding surface of the film, a portion corresponding to a starting end of the channel that comes into contact with the liquid introduction portion is subjected to hydrophobization treatment.

4. The microchip according to any one of claims 1 to 3, wherein
the substrate has a recess serving as a reaction portion at a part of a groove serving as the channel, and
on a substrate-bonding surface of the film,
a reagent is coated on a portion corresponding to the reaction portion, a portion corresponding to a starting end of a downstream channel that comes into contact with the reagent coated portion is subjected to hydrophobization treatment, and the remaining part of the portion corresponding to the channel is subjected to hydrophilization treatment.

5. The microchip according to any one of claims 1 to 4, wherein the liquid sample is an aqueous solution or an aqueous dispersion.

6. The microchip according to any one of claims 1 to 5, wherein the hydrophilization treatment is a treatment by one or more selected from application of a hydrophilization reagent, a plasma treatment, and an excimer treatment.

7. The microchip according to any one of claims 2 to 6, wherein the hydrophobization treatment is a treatment by one or more selected from application of a hydrophobization reagent and fluorine membrane coating by a plasma treatment.

8. The microchip according to any one of claims 1 to 7, wherein
the substrate is any of plastic, silicone, and glass.

9. The microchip according to any one of claims 1 to 8, wherein
the film is cycloolefin polymer (COP), cycloolefin copolymer (COC), polymethyl methacrylate (PMMA), polystyrene (PS), polycarbonate (PC), or polyethylene terephthalate (PET).

10. The microchip according to any one of claims 1 to 9, wherein
an adhesive agent or a gluing agent is applied to a region, excluding the groove and the penetrating hole, of a film-bonding surface of the substrate, and
the substrate is attached to the film by the adhesive agent or the gluing agent.

11. A method of producing a microchip for analyzing component in a liquid sample by allowing the liquid sample to pass through an internally formed channel, the method comprising:
a step of preparing a substrate comprising a bonding surface provided with a groove that forms the channel and a penetrating hole serving as a liquid introduction portion;
a step of applying an adhesive agent or a gluing agent to a region, excluding the groove and the penetrating hole, of the bonding surface;
a step of preparing a film that is to be attached to the bonding surface of the substrate, and in which at least a part of a portion corresponding to the channel has been subjected to hydrophilization treatment; and
a step of attaching the film to the bonding surface of the substrate so that the groove of the substrate is covered with the film to form the channel.

12. A method of producing a microchip for analyzing component in a liquid sample by allowing the liquid sample to pass through an internally formed channel, the method comprising:
a step of preparing a substrate comprising a bonding surface provided with a groove that forms the channel and a penetrating hole serving as a liquid introduction portion;
a step of applying an adhesive agent or a gluing agent to a region, excluding the groove and the penetrating hole, of the bonding surface;
a step of preparing a film that is to be attached to the bonding surface of the substrate, and in which a part of a portion corresponding to the channel has been subjected to hydrophilization treatment and another part corresponding to the channel has been subjected to hydrophobization treatment; and
a step of attaching the film to the bonding surface of the substrate so that the groove of the substrate is covered with the film to form the channel.

13. A method of producing a microchip for analyzing component in a liquid sample by allowing the liquid sample to pass through an internally formed channel, the method comprising:
a step of preparing a substrate comprising a bonding surface provided with a groove that forms the channel, a recess serving as a reaction portion disposed in a portion of the groove, and a penetrating hole serving as a liquid introduction portion;
a step of applying an adhesive agent or a gluing agent to a region, excluding the groove, the recess, and the penetrating hole, of the bonding surface;
a step of preparing a film that is to be attached to the bonding surface of the substrate, and in which a reagent has been coated on a portion corresponding to the reaction portion, a portion corresponding to a starting end of a downstream channel that comes into contact with the reagent-coated portion has been subjected to hydrophobization treatment, and a portion corresponding to another region of the channel has been subjected to hydrophilization treatment; and
a step of attaching the film to the bonding surface of the substrate so that the groove of the substrate is covered with the film to form the channel.
